(19) **Europäisches Patentamt / European Patent Office / Office européen des brevets**

(11) **EP 1 676 522 B1**

(12) **EUROPEAN PATENT SPECIFICATION**

(45) Date of publication and mention
of the grant of the patent:
**02.07.2008 Bulletin 2008/27**

(51) Int Cl.:
*A61B 1/05* (2006.01)　　*A61B 5/06* (2006.01)
*A61B 5/07* (2006.01)

(21) Application number: **05112932.8**

(22) Date of filing: **23.12.2005**

(54) **System for locating an in-vivo signal source**

System zur Lokalisation einer in-Vivo Signalquelle

Système pour localiser une source de signale in-vivo

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **30.12.2004 US 639964 P**

(43) Date of publication of application:
**05.07.2006 Bulletin 2006/27**

(73) Proprietor: **Given Imaging Ltd.
20692 Yoqneam (IL)**

(72) Inventor: **Horn, Eli
26316 Kiryat Motzkin (IL)**

(74) Representative: **Harrison Goddard Foote
Belgrave Hall
Belgrave Street
Leeds LS2 8DD (GB)**

(56) References cited:
**EP-A- 0 667 115　　　　WO-A-00/22975
US-A1- 2003 073 935**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates to the field of in-vivo sensing. More specifically, the present invention relates to devices, systems, and methods for locating an in-vivo signal source.

**BACKGROUND OF THE INVENTION**

Devices, systems and methods for in-vivo sensing of passages or cavities within a body, and for sensing and gathering information (e..g., image information, pH information, temperature information, electrical impedance information, pressure information, etc.), are known in the art.

In-vivo sensing devices such as capsules may include a sensing system and a transmission system, wherein the sensing system collects data and the transmission system transmits the collected data using Radio Frequency (RF) to an external receiver system, e.g., for further processing and display.

Some in-vivo imaging systems include an image sensor carried within a swallowable device such as a capsule. The in-vivo imaging device may capture and transmit images of the GI tract, or other body lumen or body cavity being imaged, while the device may pass through the entire digestive tract and may operate as an autonomous video endoscope.

Prior attempts have been made at tracking an intra-gastric and intrauterine transmitting device include spatially scanning a non-ambulatory patient with a receiver. The receiver and scanning system may locate the points with the highest reception and plots a track of the device, the assumption being that the capsule may be at the location where the strongest signal may have been received. Such systems may require laboratory device that may not be portable and may not be commercial.

Other attempts at locating an in-vivo capsule or device may analyze the statistics of signal variation during the passage of an in-vivo device, for example, through the GI tract. Large signal level variations may be observable during the passage of the capsule through specific significant locations in the lumen, and these variations may be associated with specific anatomical features. This method may be inherently inaccurate, for example, since the anatomically significant locations of the GI tract are not rigidly attached to a fixed frame of reference.

A system for tracking a swallowable capsule using an antenna array and a signal strength detector is known from EP-0667115.

**SUMMARY OF THE INVENTION**

Some embodiments of the invention provide, for example, a system and method for tracking an in-vivo image sensor, the system including a location detecting unit to locate the in-vivo image sensor over time and a data modifying unit to modify the data sampled by the location detecting based on information sensed by the in-vivo image sensor. In some embodiments of the present invention a motility detector may unit may be included and may be used to compare image data and based on that comparison, data sampled by the location detecting unit may be modified or enhanced. In other embodiments median filtering may be used to enhance data sampled by the location detecting unit. Other suitable methods may be used to modify and/or enhance data sampled form the location detection unit as may be described herein. In some embodiments, for example, the enhancement process or scheme may be performed in substantially real time and while said in-vivo signal source is in-vivo.

In some embodiments, the system may be adapted to perform other operations, for example, displaying, storing, or otherwise processing the enhanced localization data.

Embodiments of the invention may allow various other benefits, and may be used in conjunction with various other applications.

**BRIEF DESCRIPTION OF THE DRAWINGS**

The subject matter regarded as the invention is particularly pointed out and distinctly claimed in the concluding portion of the specification. The invention, however, both as to organization and method of operation, together with objects, features and advantages thereof, may best be understood by reference to the following detailed description when read with the accompanied drawings in which:

Figures 1A and 1B are schematic illustrations of a patient wearing an antenna array according to an embodiment of the invention;
Figure 2 is a schematic block diagram of a data recorder in accordance with an embodiment of the invention;
Figure 3 is a schematic block diagram of an in-vivo signal source in accordance with an embodiment of the invention;

Figure 4 is a schematic illustration of a torso surrounded by an antenna array belt in accordance with an embodiment of the invention and an estimated point of a signal source;

Figure 5 is a schematic illustration of three signal vectors in a two dimensional plane, in accordance with an embodiment of the invention;

Figure 6 is a schematic illustration of a three signal vectors in three dimensional space, in accordance with an embodiment of the invention;

Figure 7 is a schematic illustration of a graph of a weighing function for signal vectors, in accordance with an embodiment of the invention;

Figure 8 is a schematic block diagram of an in-vivo sensing system in accordance with an embodiment of the invention;

Figure 9A is a schematic illustration of a graph indicating an X-axis location of an in-vivo signal source as a function of time, in accordance with an embodiment of the invention;

Figure 9B is a schematic illustration of a graph indicating a Y-axis location of an in-vivo signal source as a function of time, in accordance with an embodiment of the invention; and

Figure 10 is a flow-chart diagram of a method of processing data points sampled by a location detecting unit to locate an in-vivo signal source, for example, an in-vivo image sensor over time in accordance with an embodiment of the present invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

## DETAILED DESCRIPTION OF THE INVENTION

In the following description, various aspects of the invention will be described. For purposes of explanation, specific configurations and details are set forth in order to provide a thorough understanding of the invention. However, it will also be apparent to a person skilled in the art that the invention may be practiced without the specific details presented herein. Furthermore, well-known features may be omitted or simplified in order not to obscure the invention.

It should be noted that although a portion of the discussion may relate to in-vivo imaging devices, systems, and methods, the present invention is not limited in this regard, and embodiments of the present invention may be used in conjunction with various other in-vivo sensing devices, systems, and methods. For example, some embodiments of the invention may be used, for example, in conjunction with in-vivo sensing of pH, in-vivo sensing of temperature, in-vivo sensing of pressure, in-vivo sensing of electrical impedance, in-vivo detection of a substance or a material, in-vivo detection of a medical condition or a pathology, in-vivo acquisition or analysis of data, and/or various other in-vivo sensing devices, systems, and methods.

It is noted that discussions herein utilizing terms such as "processing", "computing", "calculating", "determining", or the like, refer to the action and/or processes of a computer or computing system, or similar electronic computing device or platform, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. Such apparatus may be specially constructed for the desired purposes, or it may comprise a general purpose computer selectively activated, adapted, operated, configured or re-configured by a computer program stored in the computer. Such a computer program may be stored in a computer readable storage medium, such as, but not limited to, a disk, a hard disk drive, a floppy disk, an optical disk, a CD-ROM, a DVD, a magnetic-optical disk, Read-Only Memory (ROM), Random Access Memory (RAM), Electrically Programmable ROM (EPROM), Electrically Erasable and Programmable ROM (EEPROM), Flash memory, volatile memory, non-volatile memory, magnetic or optical cards, or any other type of storage media or storage unit suitable for storing electronic instructions and capable of being operatively connected to a computer system bus or a computing platform.

The processes and displays presented herein are not inherently related to any particular computer or other apparatus. Various general purpose systems may be used with programs in accordance with the teachings herein, or it may prove convenient to construct a more specialized apparatus to perform a desired method. The desired structure for a variety of these systems will appear from the description below. In addition, embodiments of the present invention are not described with reference to any particular programming language. It will be appreciated that a variety of programming languages may be used to implement the teachings of the inventions as described herein.

Some embodiments of the present invention are directed to a typically swallowable in-vivo device, e.g., a typically swallowable in-vivo sensing or imaging device. Devices according to embodiments of the present invention may be similar to embodiments described in United States Patent Application Number 09/800,470, entitled "Device and System for In-vivo Imaging", filed on 8 March, 2001, published on November 1, 2001 as United States Patent Application Publication Number 2001/0035902, and/or in United States Patent Number 5,604,531 to Iddan et al., entitled "In-Vivo Video Camera System", and/or in United States Patent Application Number 10/046,541, filed on January 16, 2002, published on August 15, 2002 as United States Patent Application Publication Number 2002/0109774. An external receiver/recorder unit, a processor and a monitor, e.g.., in a workstation, such as those described in the above publications, may be suitable for use with some embodiments of the present invention. Devices and systems as described herein may have other configurations and/or other sets of components. For example, the present invention may be practiced using an endoscope, needle, stent, catheter, etc. Some in-vivo devices may be capsule shaped, or may have other shapes, for example, a peanut shape or tubular, spherical, conical, or other suitable shapes.

Some embodiments of the present invention may be used, for example, in conjunction with devices and/or systems described in United States Patent Application Number 11/073,633, entitled "Array System and Method for Locating an In Vivo Signal Source", filed on March 8, 2005, published on July 7, 2005 as United States Patent Application Publication Number 2005/0148816; and or in conjunction with devices and/or systems described in United States Patent Number 6,904,308, entitled "Array System and Method for Locating an In Vivo Signal Source".

Embodiments of the in-vivo device are typically autonomous and are typically self-contained. For example, the in-vivo device may be or may include a capsule or other unit where all the components are substantially contained within a container, housing or shell, and where the in-vivo device does not require any wires or cables to, for example, receive power or transmit information. The in-vivo device may communicate with an external receiving and display system to provide display of data, control, or other functions, For example, power may be provided by an internal battery or a wireless receiving system. Other embodiments may have other configurations and capabilities. For example, components may be distributed over multiple sites or units. Control information may be received from an external source.

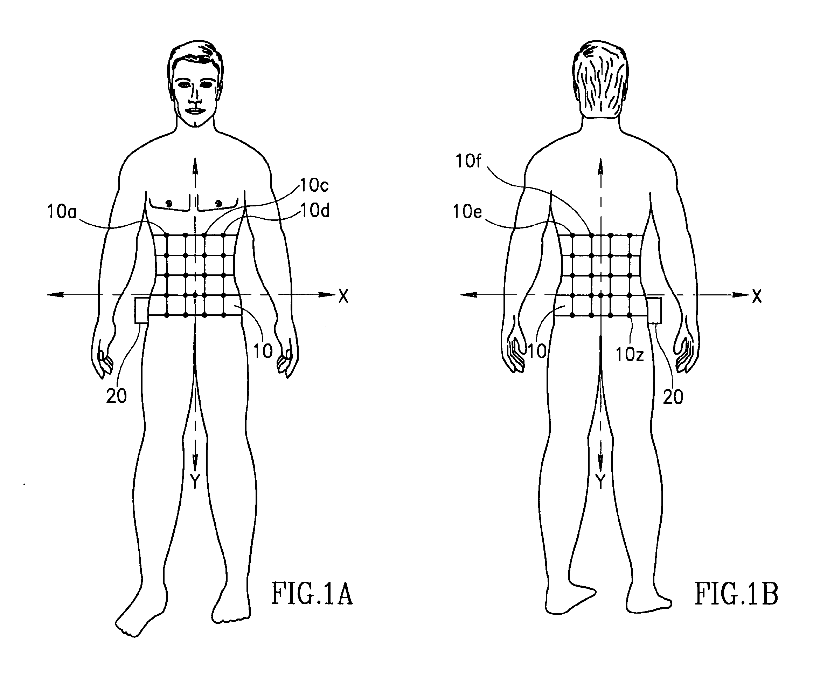

Figs. 1A and 1B schematically illustrate a patient wearing an antenna array according to an embodiment of the present invention. According to an aspect of the present invention, an in-vivo signal source, for example, an in-vivo image sensor may be located or localized using a portable or wearable antenna array or antenna array belt 10, as shown in Figs. 1A and 1B. In other embodiments the antenna array may be integral to a jacket that the patient may wear. The antenna array belt 10 may be fitted such that it may be wrapped around a patient and attached to a signal recorder 20. Additional embodiments include, for example, antenna elements having adhesive, which may adhere the element to a point on a body. Each of the antennas elements 10a through 10z in the array may connect via coaxial cables to a connector, which may connect to the recorder 20. Each antenna element 10a through 10z may be a loop antenna, a dipole antenna, or may be another suitable antenna configuration.

In one embodiment, the antenna array belt 10 may include, for example, 1 to eight antenna elements that may be typically positioned on a patient's midsection. For example, the eight antenna elements can be positioned as follows: a first antenna element may be positioned approximately on the intersection of the right seventh intercostal space and right mid clavicular line; a second antenna element may be positioned approximately on the xiphoid process; a third antenna element may be positioned approximately on the intersection of the left 7th intercostal space and left mid clavicular line; a fourth antenna element may be positioned approximately on the right lumbar region at umbilical level; a fifth antenna element may be positioned approximately above the navel; a sixth antenna element may be positioned approximately on the left lumbar region at umbilical level; a seventh antenna element may be positioned approximately on the right mid-linguinal region; and an eighth antenna element may be positioned approximately on the left mid-linguinal region. Other antenna positions and other numbers of antennas may be used in accordance with embodiments of the invention. For example, an antenna array may be positioned on a patient's back. In some embodiments, only one antenna may be used.

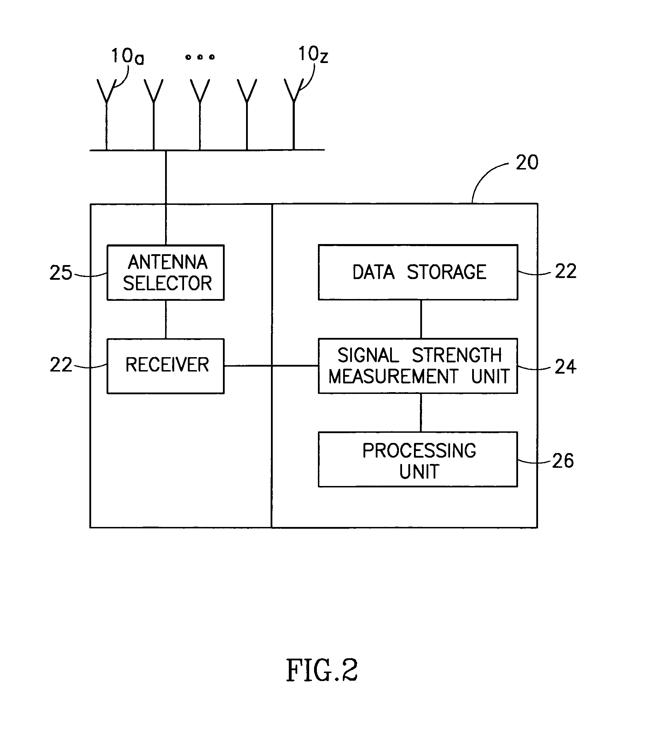

Fig. 2 schematically illustrates a data recorder 20 according to an embodiment of the present invention. Data recorder 20 may include, for example, a data storage unit 22, a receiver 21, a signal strength measurement unit and/or a signal strength detector 24, a processing unit 26, and an antenna selector 25. In alternate embodiments, the data recorder 20 may include other combinations of components, and the components described may be divided among several or other units.

In some embodiments of the present invention, the antenna array may include a plurality of antennas wherein the antennas may receive a signal and/or information from a plurality of locations, for example by an RF signal, transmitted from the in-vivo image sensor. The signal strength measurement unit 24 may measure the signal strength of signals received by the receiver 21 from a plurality of locations, for example, from each of the antenna elements 10a through 10z. The processing unit 26 may perform calculations to correlate the received signal with an estimated location of the source of the signal. The antenna selector 25 may open a signal path to single one or more antenna elements from which the receiver 21 will receive a signal. The antenna selector 25 may be adjusted to scan through all or subset of antenna elements 10a through 10z. The scan rate and pattern may be adjusted, for example, to maximize Signal to Noise Ratios (SNRs) for the received signals.

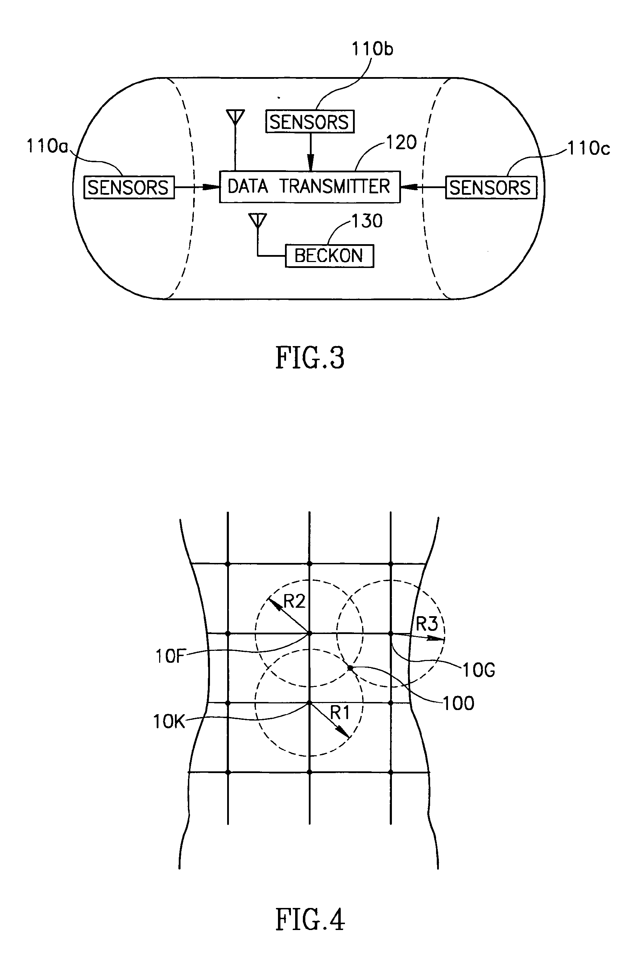

Fig. 3 schematically illustrates an in-vivo signal source 100 according to an embodiment of the present invention. In some embodiments, for example, the source 100 may be a capsule, which may be ingested. In some embodiments, the source 100 may include an in-vivo imaging or sensing device similar to an in-vivo imaging or sensing device known in the art, or may include an in-vivo imaging or sensing device having components similar to components known in the art.

The source 100 may include one or more sensors, for example, a temperature sensor 110a, a pH sensor 110b, and an image sensor or optical sensor 110c. Other sensors or sets of sensors may be used. In some embodiments, only one sensor may be included in the source 100, e.g., an imaging sensor or an image sensor. The sensors 110 may provide data, for example, to a data transmitter 120. A beacon 130 may send out an intermittent beacon signal, or the beacon 130 may be instructed or configured to transmit at or about substantially the same time the data transmitter 120 transmits a data signal. Typically, the data transmitter 120 may transmit at a higher frequency than the beacon 130, but need not. In some embodiments, the data transmitter 120 may transmit, for example, a non-modulated signal as a beacon signal. In some embodiments, a beacon and/or beacon signal need not be used.

Fig. 4 schematically illustrates a torso surrounded by an antenna array belt 10 according to an embodiment of the present invention and an estimated point of a signal source. There is shown a close-up of a human torso wearing a belt 10 or adhesive antenna array according to an embodiment of the present invention. Also visible is an estimated location of an in-vivo signal source 100. The location is shown as the intersection point of three circles having radius R1, R2 and R3, each radius value being an estimated distance value of the source 100 from each of antenna elements 10k, 10f and 10g, respectively. The distance values may be calculated by a processing unit, e.g., by processing unit 26, based on signal strength measurements preformed by signal strength measurement unit 24.

In one embodiment, for example, a propagation assumption may be used in processing the localization signal data, e.g., assuming that radiation attenuation is linear within the body. This may be equivalent to:

$$\mathrm{Ir} = \mathrm{Io} \propto \alpha * r \qquad (\text{Equation 1})$$

wherein:

r may indicate the distance (in cm) between the source 100 and the antenna;
Io may indicate the signal level (in dBm) at the source 100;
Ir may indicate the signal level (in dBm) at distance r; and
$\alpha$ may indicate an absorption coefficient (in dB/cm).

It is noted that Equation 1 above is presented for exemplary purposes, and that additional or alternate equations, functions, formulae, parameters, algorithms, assumptions and/or calculations may be used in accordance with embodiments of the invention. Other suitable signal source triangulation techniques may be used in accordance with embodiments of the invention.

In accordance with some embodiments of the invention, the assumption of linear attenuation may be valid at a working frequency range (e.g., 200-500 MHz) and at intermediate distances between the transmitter and receiver, i.e. for distances of half a wavelength to 2-2.5 wavelengths. Linear attenuation may be valid in between other frequencies and/or ranges. In some embodiments, knowing the signal level at the source 100 and the measured signal level at each antenna, the distance between the source 100 and the antenna may be derived.

The discussion herein presents yet another example of a method of locating, localizing, or estimating the location of, an in-vivo signal source according to some embodiments of the present invention.

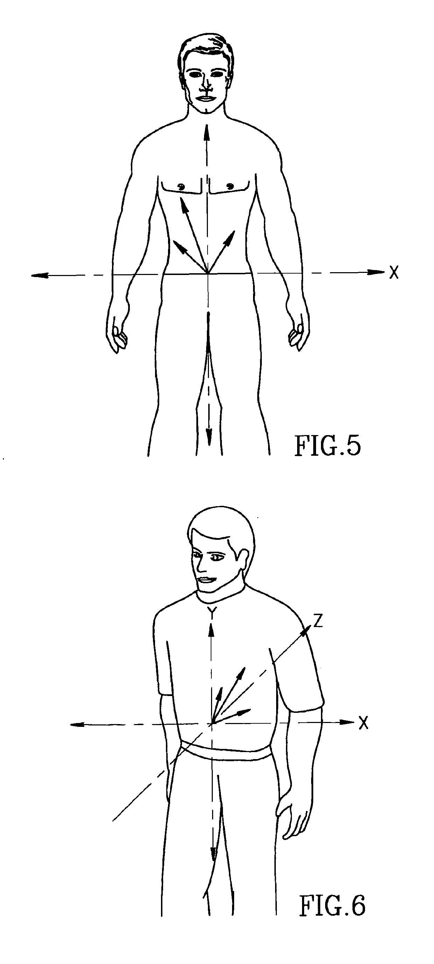

Fig. 5 schematically illustrates three signal vectors in a two dimensional plane, in accordance with an embodiment of the invention. The three signal vectors may relate to signals received at three antenna elements, for example, 10d, 10p, 10q. Beginning at the origin of a coordinate system centered at the navel, each signal vector may point in the direction of its respective antenna element, and may have a magnitude relating to the strength of the received signal.

In some embodiments, each signal vector may be calculated, for example, as the product of a pointing vector from the origin to the point where its respective antenna element is placed, multiplied by a normalized received signal value. A normalized signal strength value may be computed, for example, by dividing each measured signal strength value by the strongest measured value. This may result in the strongest measured value being normalized to 1, and the rest to values less than one. Thus, the signal vector pointing to an antenna element receiving the strongest signal level may look substantially identical to its pointing vector, and the other signal vectors may be shorter than their pointing vectors.

In accordance with some embodiments of the invention, the estimated point or location of the signal source 100 may be estimated, for example, as the vector sum of all the signal strength vectors, i.e., the location vector. In some embodiments, signal vectors may be calculated for two or more antenna elements 10a through 10z.

In some embodiments, signal vectors may be calculated for only elements placed at the front of the torso. In some embodiments, as illustrated schematically in Fig. 6, signal vectors may be calculated for elements placed at the back of the body, as shown in Fig. 1B. In Fig. 6, the point estimated to be the location of the signal source 100 is within the body. Typically, the location vector starts at the origin of a three dimensional system and ends at a point within the body.

In accordance with some embodiments of the invention, an absolute coordinate set may be used, wherein points on the body may be measured in terms of standard units, for example, centimeters or inches. In some embodiments, values may be assigned relative to anatomical points on the body, and then the results may be normalized. For example, an antenna element placed approximately at the navel may be given the coordinate set 0,0; an element placed approximately at the right end of the torso at navel level may be given the coordinate set 5,0; and an element place at left end of the torso may be given the coordinate set -5,0. Distance values or vector magnitudes may be calculated using these coordinate sets, and then the values may be proportionally adjusted to fit the body's actual dimensions. For example, if there was calculated a distance value of 2.5 inches based on the above stated coordinates, but it was later measured that the body had actually 7 inches from the navel to the right end, the distance value of 2.5 could be adjusted in the same proportion, e.g., 7/5.

In some embodiments, only one or more, e.g., two or three or four, strongest signal sources may be used, rejecting the weaker signal strength values, to calculate signal vectors or distance values upon which a location estimate may be based. Once the strongest group of signals may be identified, a second signal strength measurement may be performed. The processing unit 26 may be adapted to perform a conventional vector sum operation, for example, on a subset of the largest vectors, and to perform a weighted sum operation on the signal vectors which may be relatively smaller. Other suitable processing operations, calculations or estimations may be performed using one or more of the collected signals.

In some embodiments, the antenna selector 25 may be adjusted to perform a scan of only the antenna elements from which the strongest signals may have been received, excluding substantially all other antenna elements. In some embodiments, excluding or rejecting signal information from antennas providing weak signals, may increase Signal to Noise Ratios (SNRs).

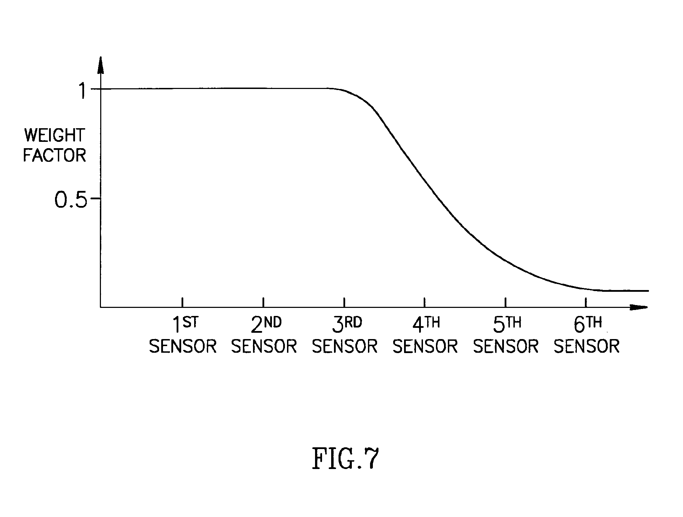

In some embodiments, location vectors or distance values may be calculated relating to many antenna elements, and signal vectors having relatively low magnitudes may be multiplied by a reducing factor or a weigh factor, e.g., as illustrated schematically in Fig. 7.

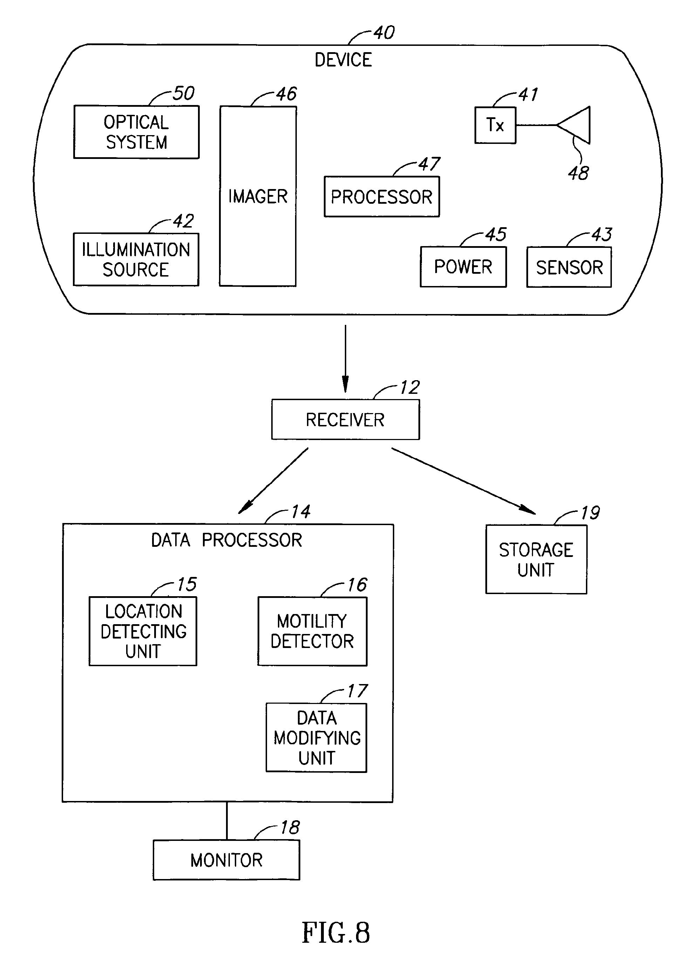

In some embodiments, an estimated location of the in-vivo signal source 100 may be tracked substantially continuously or semi-continuously, for example, by a location detecting unit 15 (Fig. 8). In some embodiments, for example, an instantaneous velocity vector for the signal source 100 may be computed, e.g., using the location information. In one embodiment, for example, the velocity vector may be the vector starting at the tip of a first location vector and ending at the tip of a consecutive location vector. In an alternate embodiment, for example, the speed of the signal source 100 may be computed as a derivative of its position, and its direction or orientation may be plotted on a display or a graph functionally associated with the data recorder 20.

It is noted that in some embodiments of the invention, a procedure for detecting a defective antenna elements may be used. For example, in some embodiment, if an antenna element may be determined to be defective, non-operational, semi-operational or malfunctioning, the entire trajectory may be invalidated. In one embodiment, for example, readings for all frames (if not discarded) may be collected, for each antenna, into two bins; for example, Bin1 having the number of readings in the range 0 to 40, and Bin2 having the number of readings in the range 41 to 255; or, for example, Bin1 having the number of readings in the range 0 to 107, and Bin2 having the number of readings in the range 108 to 255. The result may include, for example, eight histograms of two bins each, one for each antenna. In one embodiment, if Bin1/(Bin1+Bin2)>0.75 then the antenna may be determined to be defective, and otherwise the antenna may be determined to be functional. In some embodiments, the trajectory may be considered valid, for example, if all antennas are determined to be functional. Further, if the Reception(n)<60 (for the first example) or if the Reception(n)<117 (for the second example), then the current sensor readings may be discarded. The parameter 'n' may represent one of the antennas, e.g. antennas 10f, 10g, or 10k, in the antenna array.

FIG. 8 illustrates a schematic diagram of an in-vivo sensing system in accordance with an embodiment of the present invention. In one embodiment, the system may include a device 40 having an image sensor 46, an illumination source 42, a power source 45, and a transmitter 41. Device 40 may be an example of signal source 100 of Fig. 3. In some embodiments, device 40 may be implemented using a swallowable capsule, but other sorts of devices or suitable implementations may be used. Outside a patient's body may be, for example, an image receiver 12 (including, for example, an antenna or an antenna array), a storage unit 19, a data processor 14, and a monitor 18. Data processor 14 may include a location detecting unit to detect and/or to construct, for example, a two dimensional tracking curve, for example, in substantially real time, of the location of device 40, for example, an in-vivo image sensor, over time as may be described herein. In other embodiments of the present invention, a three dimensional tracking curve may be constructed to track the location of the in-vivo sensing unit. According to some embodiments of the present invention, data processor 14 may include a data modifying unit 17 that may modify, for example, enhance at least some of the data obtained from location detecting unit 15. According to one embodiment, data processor 14 may include a motility detector 16 to detect, for example, if device 40 may be in motion at a given time and the data modifying unit 17 may, for example, enhance or modify data points sampled by the location detecting unit 15, for example, in substantially real time, as may be described herein. The motility detector 16 may for example compare image frames and/or data from image frames captured from device 40 in order to determine if device 40 advanced between capturing of frames, other methods of determining motility, for example, as a function of time may be implemented, for example by using sensors other than image sensors or using data from, for example, more than one sensor. In some embodiments of the present invention, the motility detector 16 may be integral to the data modifying unit 17. Other suitable methods of incorporating a location detecting unit 15, a motility detector 16, and a data modifying unit 17 may be implemented. For example motility detector 16 may be included in data modifying unit 17. Other suitable arrangements may be used.

Transmitter 41 may operate using radio waves; but in some embodiments, such as those where device 40 may be or may be included within an endoscope, transmitter 41 may transmit data via, for example, wire, optical fiber and/or other suitable methods.

Device 40 typically may be or may include an autonomous swallowable capsule, but device 40 may have other shapes and need not be swallowable or autonomous. Embodiments of device 40 may be typically autonomous, and may be typically self-contained. For example, device 40 may be a capsule or other unit where all the components may be substantially contained within a container or shell, and where device 40 may not require any wires or cables to, for example, receive power or transmit information.

In some embodiments, device 40 may communicate with an external receiving and display system 18 (e.g., through receiver 12) to provide display of data, control, or other functions. In embodiments of the present invention, power may be provided to device 40 using an internal battery, an internal power source, or a wireless system to receive power. Other embodiments may have other configurations and capabilities. For example, components may be distributed over multiple sites or units, and control information may be received from an external source.

In one embodiment, device 40 may include an in-vivo video camera, for example, image sensor 46, which may capture and transmit images of, for example, the GI tract while device 40 may pass through, for example, the GI lumen. Other lumens and/or body cavities may be imaged and/or sensed by device 40. In some embodiments, image sensor 46 may include, for example, a Charge Coupled Device (CCD) camera or image sensor, a Complementary Metal Oxide Semiconductor (CMOS) camera or image sensor, a digital camera, a stills camera, a video camera, or other suitable image sensors, cameras, or image acquisition components.

In one embodiment, image sensor 46 in device 40 may be operationally connected to transmitter 41. Transmitter 41 may transmit images to, for example, image receiver 12, which may send the data to data processor 14 and/or to storage unit 19. Transmitter 41 may also include control capability, although control capability may be included in a separate component. Transmitter 41 may include any suitable transmitter able to transmit image data, other sensed data, and/or other data (e.g., control data) to a receiving device. For example, transmitter 41 may include an ultra low power Radio Frequency (RF) high bandwidth transmitter, possibly provided in Chip Scale Package (CSP). Transmitter 41 may transmit via antenna 48. Transmitter 41 and/or another unit in device 40, e.g., a controller or processor 47, may include control capability, for example, one or more control modules, processing module, circuitry and/or functionality for controlling device 40, for controlling the operational mode or settings of device 40, and/or for performing control operations or processing operations within device 40.

Power source 45 may include one or more batteries. For example, power source 45 may include silver oxide batteries, lithium batteries, other suitable electrochemical cells having a high energy density, or the like. Other suitable power sources may be used. For example, power source 45 may receive power or energy from an external power source (e.g., a power transmitter), which may be used to transmit power or energy to device 40.

In some embodiments, power source 45 may be internal to device 40, and/or may not require coupling to an external power source, e.g., to receive power. Power source 45 may provide power to one or more components of device 40 continuously, substantially continuously, or in a non-discrete manner or timing, or in a periodic manner, an intermittent manner, or an otherwise non-continuous manner. In some embodiments, power source 45 may provide power to one or more components of device 40, for example, not necessarily upon-demand, or not necessarily upon a triggering event or an external activation or external excitement.

Optionally, in one embodiment, transmitter 41 may include a processing unit or processor or controller, for example, to process signals and/or data generated by image sensor 46. In another embodiment, the processing unit may be implemented using a separate component within device 40, e.g., controller or processor 47, or may be implemented as an integral part of image sensor 46, transmitter 41, or another component, more than one component, or may not be needed. The optional processing unit may include, for example, a Central Processing Unit (CPU), a Digital Signal Processor (DSP), a microprocessor, a controller, a chip, a microchip, a controller, circuitry, an Integrated Circuit (IC), an Application-Specific Integrated Circuit (ASIC), or any other suitable multi-purpose or specific processor, controller, circuitry or circuit. In one embodiment, for example, the processing unit or controller may be embedded in or integrated with transmitter 41, and may be implemented, for example, using an ASIC.

In some embodiments, device 40 may include one or more illumination sources 42, for example one or more Light Emitting Diodes (LEDs), "white LEDs", or other suitable light sources. Illumination sources 42 may, for example, illuminate a body lumen or cavity being imaged and/or sensed. An optional optical system 50, including, for example, one or more optical elements, such as one or more lenses or composite lens assemblies, one or more suitable optical filters, or any other suitable optical elements, may optionally be included in device 40 and may aid in focusing reflected light onto image sensor 46 and/or performing other light processing operations.

In some embodiments, the components of device 40 may be enclosed within a housing or shell, e.g., capsule-shaped, oval, or having other suitable shapes. The housing or shell may be substantially transparent or semi-transparent, and/or may include one or more portions, windows or domes which may be substantially transparent or semi-transparent. For example, one or more illumination source(s) 42 within device 40 may illuminate a body lumen through a transparent or semi-transparent portion, window or dome; and light reflected from the body lumen may enter the device 40, for example, through the same transparent or semi-transparent portion, window or dome, or, optionally, through another transparent or semi-transparent portion, window or dome, and may be received by optical system 50 and/or image sensor 46. In some embodiments, for example, optical system 50 and/or image sensor 146 may receive light, reflected from a body lumen, through the same window or dome through which illumination source(s) 42 illuminate the body lumen.

In some embodiments, image sensor 46 may acquire in-vivo images continuously, substantially continuously, or in a non-discrete manner, for example, not necessarily upon-demand, or not necessarily upon a triggering event or an external activation or external excitement; or in a periodic manner, an intermittent manner, or an otherwise non-continuous manner.

In some embodiments, transmitter 41 may transmit image data continuously, or substantially continuously, for example, not necessarily upon-demand, or not necessarily upon a triggering event or an external activation or external excitement; or in a periodic manner, an intermittent manner, or an otherwise non-continuous manner.

Data processor 14 may analyze the data received via receiver 12 from device 40, and may be in communication with storage unit 19, e.g., transferring frame data to and from storage unit 19. Data processor 14 may also provide the analyzed data to monitor 18, where a user (e.g., a physician) may view or otherwise use the data. In one embodiment, data processor 14 may be configured for real time processing and/or for post processing to be performed and/or viewed at a later time. In the case that control capability (e.g., delay, timing, etc) may be external to device 40, a suitable external device (such as, for example, data processor 14 or image receiver 12) may transmit one or more control signals to device 40.

Monitor 18 may include, for example, one or more screens, monitors, or suitable display units. Monitor 18, for example, may display one or more images or a stream of images captured and/or transmitted by device 40, e.g., images of the GI tract or of other imaged body lumen or cavity. Additionally or alternatively, monitor 18 may display, for example, tracking data, for example, in a least two dimensions, of the in-vivo sensor, control data, location or position data (e.g., data describing or indicating the location or the relative location of device 40), orientation data, and various other suitable data. In one embodiment, for example, both an image and its position or location may be presented using monitor 18 and/or may be stored using storage unit 19. Other systems and methods of storing and/or displaying collected image data and/or other data may be used.

In some embodiments, in addition to or instead of revealing pathological or other conditions of the GI tract, the system may provide information about the location of these conditions. Suitable tracking devices and methods are described in embodiments of the above-mentioned United States Patent Number 5,604,531 and/or United States Patent Application Number 10/150,018, filed on May 20, 2002, entitled "Array System and Method for Locating an In-Vivo Signal Source", published on November 21, 2002 as United States Patent Application Publication Number 2002/0173718, assigned to the common assignee of the present invention. Other suitable location identification systems and methods may be used in accordance with embodiments of the present invention.

Typically, device 40 may transmit image information in discrete portions. Each portion may typically correspond to an image or a frame and/or may correspond to a few lines of image data; other suitable transmission methods may be used. For example, in some embodiments, device 40 may capture and/or acquire an image once every half second, and may transmit the image data to receiver 12. Other constant and/or variable capture rates and/or transmission rates may be used.

Typically, the image data recorded and transmitted may include digital color image data; in alternate embodiments, other image formats (e.g., black and white image data) may be used. In one embodiment, each frame of image data may include 256 rows, each row may include 256 pixels, and each pixel may include data for color and brightness according to known methods. According to other embodiments, a 320 by 320 pixels image sensor may be used. Pixel size may be, for example, between 5 to 6 microns. According to some embodiments, pixels may be each fitted with a micro lens

For example, in each pixel, color may be represented by a mosaic of four subpixels, each sub-pixel corresponding to primaries such as red, green, or blue (where one primary, e.g., green, may be represented twice). The brightness of the overall pixel may be recorded by, for example, a one byte (e.g., 0-255) brightness value. In one embodiment, for example, image data may be represented using an array of 64 by 64 pixels or super-pixels or boxes, each including data indicating values for red, green (repeated twice) and blue. Other suitable data formats may be used, and other suitable numbers or types of rows, columns, arrays, pixels, sub-pixels, boxes, super-pixels and/or colors may be used.

Optionally, device 40 may include one or more sensors 43, instead of or in addition to a sensor such as image sensor 46. Sensor 43 may, for example, sense, detect, determine and/or measure one or more values of properties or characteristics of the surrounding of device 40. For example, sensor 43 may include a pH sensor, a temperature sensor, an electrical conductivity sensor, a pressure sensor, or any other known suitable in-vivo sensor.

It is noted that since in-vivo device 40 may be an example of signal source 100, portions of the discussion herein relating to signal source 100 relate also to device 40, and vice versa. Furthermore, although a portion of the discussion herein relates, for exemplary purposes, to X, Y and/or Z dimensions, axes or vectors, and/or to vertical or horizontal dimensions or locations, the invention is not limited in this regard; the dimensions, directions, locations, axes and/or vectors may be relative, and in some embodiments, directions, directions, locations, axes and/or vectors may be swapped or exchanged, or other coordinate systems may be used.

In accordance with some embodiments of the invention, enhancement or alteration of localization and/or location data may be performed using, for example, data collected by or transmitted by an in-vivo device (e.g., device 40 or signal source 100), for example, data and/or information separate from location data itself For example, location data may be inherent in a signal sent by the in-vivo device, or may be in a beacon sent by the in-vivo device, while other and additional data such as sensing data (e.g., image data, pH data, etc.) may be sent separately from location data. In one embodiment, sensing data may be considered non-location data collected by the in-vivo device 40. In some embodiments, location data may be inherent in a data signal that may primarily contain sensed data. In some embodiments of the present invention, more than one, for example two, possibly independent types of sensed data may be used to determine location and/or change in location. For example, signal strength picked up from an in-vivo transmitting device 40 at one or more antennas as well as an image frame stream captured by the in-vivo device 40 may be used to determine location, tracking curve and/or change in location of an in-vivo device 40. In such an embodiment, the signal strength picked up may be the signal strength of the image frame stream captured by the in-vivo device 40 and received by more than one antenna. In one example, comparison of subsequent image frames may be instrumental in either confirming or refuting a change in the location of the in-vivo device 40 that may have been calculated based on the array of signal strengths over more than one antenna. As such both received signal strength as well as image data may be used to determine the location, change in location, or location curve, and/or tacking curve of an in-vivo device 40. In other embodiments data other than image data and/or signal strength data may be used to determine location and/or change in location and other data may be used to confirm and/or refute a change in location of an in-vivo device 40 determined based on one or more streams of data. For example, temperature, pH, acceleration, oxygen saturation, or other sensed data sensed in-vivo may be used to determine location and/or change of location of an in-vivo device 40. For example, sensed data transmitted out of the body and received by multiple antennas may be used together with the data corresponding to and/or carried on the received signal strength at one or more of the multiple antennas to determine the tracking curve, location of the vivo device 40, and/or motility. In one embodiment, sensed data may determine and/or identify the body lumen within which the in-vivo device 40 may be located, for example in a specific lumen of the GI tract, e.g. esophagus, stomach, small intestine, large intestine, etc. Information regarding the lumen may help characterize the expected movement of the in-vivo device 40 in the identified lumen. For example, if an in-vivo device 40 may be currently located in the stomach area as may be determined based a pH sensor readings or other sensors readings (e.g. more than one sensor reading), the capsule may be expected to tumble and move in for example random directions. The tracking algorithm in this case may be adjusted, for example, to filter random motion in the displayed localization and/or tracking curve. Other suitable adjustments to the localization algorithm may be made based one or more types of sensed data. In other body lumens, for example, in the small intestine the in-vivo device 40 may be expected to advance in a more orderly manner. Independent information of this caliber may aid in increasing the coherency and/or usability of the localization data. In another example, knowledge of the body lumen within which the in-vivo device 40 may be located may help determine one or more specific directions that the capsule may be moving in. For example, through the esophagus most of the movement may be expected in a specific plane, for example in the X-Y plane, or some other plane, and sharp changes in for example the Z direction may be attributed to noise, for example. Other methods and other signals and/or data may be used to increase the coherency of the tracking curve of an in-vivo device 40. The in-vivo device 40 may be located in body lumens other than the GI lumens. Other methods of performing fusion of multiple data sources may be used to determine or improve location and/or motility information of the in-vivo device 40.

In some embodiments of the present invention, the original location data may indicate that, for example, the device 40 may have been displaced, for example between two consecutive sampling points, a distance that may be assumed to be larger than may be considered probable or possible, for example, for a given region. For example, one sampled point may indicate that device 40 may be in a location A and a subsequent sampled data point, sampled after, for example, one sampling period may indicate that device 40 may be in a location B. In one example, the distance between location A, a previous data point, and location B, a current data point, may be larger than may be assumed probably or possible for device 40 to move during, for example, a single sample period. In one embodiment of the present invention, a current data point may be modified if its distance from a previous data point may be above a pre-determined threshold. In one embodiment of the invention, a set and/or plurality of data point that may indicate displacement of the device 40 over a pre-determined threshold, the current data point, for example, sampling point B in the above example, may be repositioned to correspond to a displacement equaling to, for example, the pre-determined threshold, or other pre-determined value. The new position of the sampled data may be placed in the same relative direction of the original sampled point, for example sampled point B in the above example. As such the localization curve may be modified to eliminate substantially improbable displacements of the device 40.

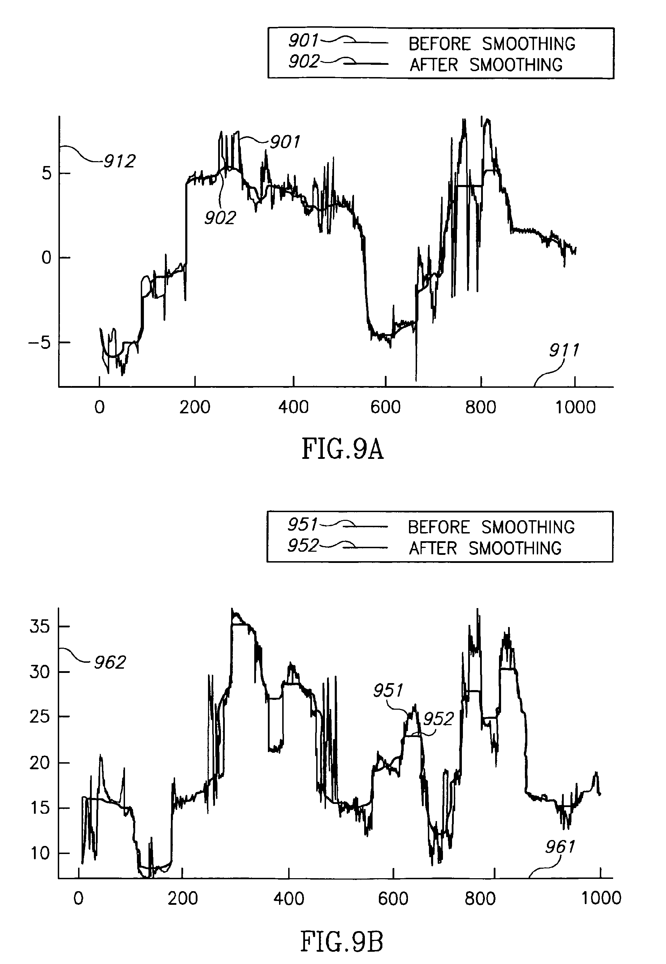

In accordance with some embodiments of the invention, smoothing or filtering of localization data in one or more dimensions, for example in at least two dimensions may be performed, for example, in substantially real time. Reference is now made to Fig. 9A and 9B schematically illustrating a graph indicating for example an X-axis location (e.g., horizontal location) or an Y-axis location (e.g. vertical axis) of a sample signal source 100, for example, and image sensor as a function of and/or over time obtained from, for example a location detecting unit 15, the same sample signals after applying a median filter to reduce noise. Fig. 9A and 9B may be representative of other suitable axis or dimensions besides or in addition to the X-axis and Y-axis. In some embodiments of the present invention and typically, median filtering may be included in data modifying unit 17 and may be performed in, for example, real time. In other embodiments, median filtering may included in other suitable units.

Referring to Fig. 9A, a horizontal axis 911 may indicate, for example, image frame number, time units, or received data packets or other data. For example, a marking "400" on the horizontal axis 911 may indicate that 400 frames were received by recorder 20 or receiver 12. This may indicate, for example, that 200 seconds elapsed, if frames may be transmitted by signal source 100 at a rate of, for example, two frames per second.

A vertical axis 912 may indicate, for example, an X-axis location (e.g., a horizontal location) of signal source 100. For example, the marking "5" on the vertical axis 912 may indicate an X-axis location of 5 centimeters, wherein a pre-defined location (e.g., approximately over the navel) may be pre-defined as having a "0" X-axis value. Other measurement units may be used, and other points of reference may be used. Normalization may be applied so to the horizontal and/or vertical axis or other suitable units may be used.

In accordance with some embodiments of the invention, a graph 901 may represent the X-axis location of signal source 100 in-vivo as a function of frame numbers or elapsed time. In some embodiments, graph 901 may be enhanced, corrected, refined, modified or otherwise processed, for example, to allow more-reliable tracking of signal source 100 and to eliminate or decrease potential inaccuracies. Such enhancement or processing may be performed, for example, by data modifying unit 17, by recorder 20, by processing unit 26, by receiver 12 or by data processor 14, or by another suitable unit. In some embodiments, the enhancement or processing may include, for example, smoothing of graph 901 and/or of data presentable using graph 901, e.g., using linear smoothing, using average smoothing, using non-linear smoothing, for example using median smoothing or filtering. In one embodiment, for example, data representing X-axis location of signal source 100 may be subject to median smoothing or median filtering, and graph 901 may be modified or processed to result in an enhanced graph, e.g., a graph 902. In some embodiments of the present invention, median filtering may be applied to preserve sharp transitions that may be inherent in motility of device 40 while filtering out noise. The results of the median smoothing may be further used, for example, to display or store enhanced localization data of signal source 100. The parameters defming the median filter or other suitable filter may be defined based on knowledge of the motility of device 40 within the body lumen. For example the degree of smoothing may be adjusted to reflect a rate at which device 40 may be expected to advance through a body lumen, so that a calculated or generated gradient or slope that may reflect a rate above which device 40 may be expected to advance may be smoothed out using one or more suitable smoothing techniques, e.g. median filters.

Referring to Fig. 9B, a horizontal axis 961 may indicate, for example, image frame number, time units, or received data packets or other data. For example, a marking "400" on the horizontal axis 961 may indicate that 400 frames were received by recorder 20 or receiver 12. This may indicate, for example, that 200 seconds elapsed, if frames may be transmitted by signal source 100, for example, at a rate of two frames per second.

A vertical axis 962 may indicate, for example, a Y-axis location (e.g., a vertical location) of signal source 100. For example, the marking "10" on the vertical axis 912 may indicate a Y-axis location of 10 centimeters, wherein a pre-defined location (e.g., approximately over the navel) may be pre-defined as having a "0" Y-axis value. Other measurement units may be used, and other points of reference may be used.

In accordance with some embodiments of the invention, a graph 951 may represent the Y-axis location of signal source 100 in-vivo as a function of frame numbers or elapsed time. In some embodiments, graph 951 may be enhanced, corrected, refined, modified or otherwise processed by for example, data modifying unit 17, for example, to allow a more-reliable and/or coherent localization of signal source 100 and to eliminate or decrease potential inaccuracies for example, inaccuracies due to noise or due to random movement of the capsule, e.g. change in the orientation of the capsule. Data modifying unit 17 may be integral to, for example, recorder 20, processing unit 26, receiver 12 and/or data processor 14, or by another suitable unit. In some embodiments the enhancement or processing by, for example, data modifying unit 17 may include, for example, smoothing of graph 951 and/or of data presentable using graph 951, e.g., using linear smoothing, using average smoothing, or using median smoothing. In one embodiment, for example, data representing Y-axis location of signal source 100 may be subject to median smoothing or median filtering, for example in substantially real time, and graph 951 may be modified or processed to result in an enhanced graph, e.g., a graph 952. The results of the median smoothing may be further used, for example, to display or store enhanced localization data of signal source 100.

Referring to Figs. 9A and 9B, some embodiments may use X-axis localization data or graph enhancement, Y-axis localization data or graph enhancement, or both X-axis and Y-axis localization data or graph enhancement. For example, in one embodiment, both X-axis and Y-axis localization data or graph enhancement may be subject to median smoothing or median filtering. In some embodiments, median-filtered localization data or graphs may be stored, displayed or processed, instead of or in addition to non-enhanced data.

It is noted that although a portion of the discussion herein may relate, for exemplary purposes, to an X-axis and a Y-axis, or to a horizontal location and a vertical location, the present invention is not limited in this regard. Embodiments of the invention may be used in conjunction with another axis (e.g., a Z-axis) or other suitable anises. Furthermore, such axes may be, but need not be, perpendicular to each other, or substantially parallel to a person's body or skin.

In accordance with embodiments of the invention, median filtering, median smoothing, and/or other suitable methods of filtering, smoothing or enhancing may be performed on localization signals, localization data, localization graphs, motility data, or images or visual representations corresponding to localization data. In some embodiments, the filtering, smoothing or enhancement may be performed substantially in real time, e.g., upon reception of localization signals and while the signal source 100 may be in-vivo. In alternate embodiments, the filtering, smoothing or enhancement may be performed at a later period of time, e.g., during post-processing of previously-collected localization data.

In addition to, or instead of, median filtering, median smoothing or other non-linear smoothing of localization data or graphs, other suitable data or graph enhancement methods or algorithms may be used. For example, in one embodiment, a tracking curve may have a "digitized" or jagged look when displayed, and curve smoothing (e.g., X-Z, Y-Z, and/or X-Y curve smoothing) may be applied to enhance and improve the location data. This may be performed, for example, while maintaining the relative locations of location data points on the tracking curve. It is noted that in some embodiments, smoothing of tracking curves may be different than smoothing each of two one-dimensional vector points since for example there may be no uniform spacing of the points on a two-dimensional tracking curve.

In some embodiments, location data curve smoothing (e.g., X-Z curve smoothing) may be performed by for example, data modifying unit 17, using a suitable algorithm, method or process. In one embodiment, for example, the length of the curve may be calculated or determined; and the distance of each point on the curve, relative to the start of the curve, may be determined. The values of each of two one-dimension sampled vectors may be smoothed using a suitable method, e.g., using boxcar smoothing as known in the art. Then for example, the curve may be re-sampled in a spatial plane, substantially uniformly, along the curve line. For example, the smoothed vectors may be re-sampled at the relative original positions. This may result in, for example, a data location graph having smooth curves or relatively smooth curves, which may be used for further display, storage or processing.

In accordance with some embodiments, certain location data calculated by recorder 20 based on received signals, may be over-ruled, disregarded, discarded, not used or not displayed, when one or more pre-defined conditions may be met. For example, data points sampled from the location detecting unit 15 that may indicate, for example, that signal source 100 may have moved from a first location to a second location may be disregarded when one or more pre-defined conditions may be met. In some embodiments motility of device 40 may be determined in the motility detecting unit 16 and may be used to determine the one or more pre-defined conditions. In one embodiment, for example, if a first image and a second image (e.g., two consecutive images) received from signal source 100, in-vivo device 40 or image sensor 46, are compared and determined to be identical, substantially identical or generally identical, and/or indicate non-movement of the image sensor then it may be determined that the location of signal source 100 or in-vivo-device 40 may not have changed at the time period between acquiring the first image and acquiring the second image. In some embodiments, for example, if image data collected by device 40 may indicate that device 40 may not be moving, then the location detecting unit 15 that may indicate a movement of device 40 may be over-ruled, discarded, replaced with a data point indicating non-movement of device 40, or replaced with data sampled by the location detecting unit associated with a previous location of device 40.

In some embodiments, two or more images acquired by in-vivo device 40 may be compared or otherwise analyzed, for example, by motility detector 16 in order to generate data to track device 40 in-vivo, or in order to generate analysis results which may be used to enhance or modify localization data. In some embodiments, the comparison or analysis of images, for example, as may be performed in the motility detector 16, may be in accordance with methods and algorithms known in the art, for example, as described in United States Patent Number 6,709,387, entitled "System and method for controlling in-vivo camera capture and display rate". The comparison or analysis may result in, for example, a conclusion that the in-vivo device 40 may be moving or may not be moving, and data point(s) sampled from the location detecting unit 15 may be updated in the data modifying unit 17 according to the analysis or comparison results, for example, the comparison results performed in the motility detector 16. In other embodiments motility detector 16 may implement information other or in addition to image information to detect motility of device 40.

In some embodiments, image comparison, image processing, or image analysis may be used as one of the parameters that a data modifying unit 17 may take into account. In one embodiment, the image comparison or image analysis may influence in reducing the noise of data sampled from a location detecting unit, such that an image comparison result indicating non-movement of device 40 may result in modifying the location data to correspond to such non-movement.

In some embodiments, multiple processes or operations may be used in combination, to achieve further enhancement or refinement of location and/or tracking data of signal source 100. For example, in one embodiment, non-linear smoothing, e.g median filtering may be used on data sampled from the location detecting unit 15 when device 40 may be determined to be in motion; and image comparison may be used in the motility detector 16 to determine, at a different time, for example that device 40 may not be moving and therefore data points sampled by location detecting unit may be modified to indicate such non-movement. Other suitable analysis based on other sensors may be used to enhance or determine location and/or change in location and/or tracking curve.

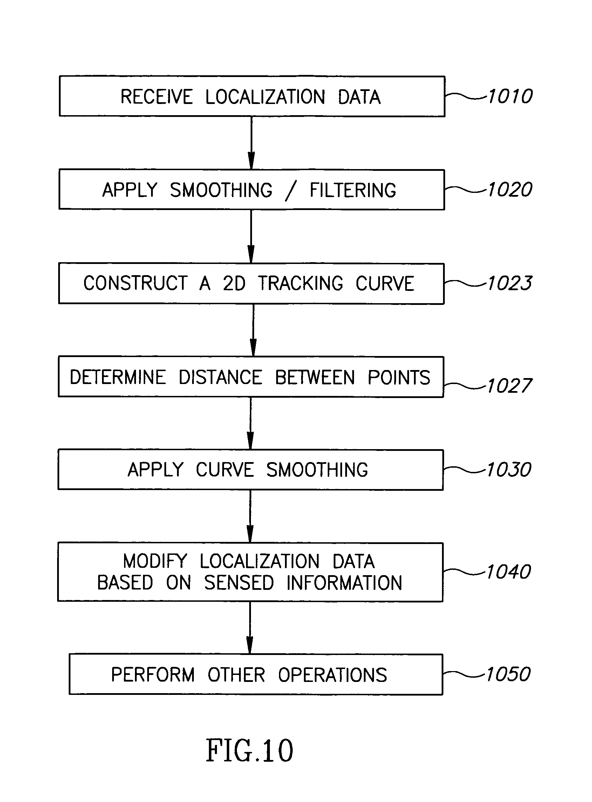

Fig. 10 is a flow-chart diagram of a method of processing data points sampled by location detecting unit 15 tracking in-vivo signal source in accordance with an embodiment of the present invention. The method of Fig. 10, as well as other suitable methods in accordance with embodiments of the invention, may be used, for example, in association with the antenna array of Figs. 1A-1B, with recorder 20 of Fig. 2, with processing unit 26 of Fig 2, with signal source 100 of Fig. 3, with device 40 of Fig. 8, with the system of Fig. 8, and/or with other suitable devices and systems for in-vivo imaging or in-vivo sensing. A method according to embodiments of the invention need not be used in an in-vivo context.

In some embodiments, as indicated at box 1010, the method includes, for example, receiving and/or sampling data points from location detecting unit 15. This may be performed, for example, by recorder 20 of Fig. 2.

As indicated at box 1020, the data modifying in data modifying unit 17 may optionally include, for example, applying a smoothing or a filtering process, for example, median filtering or other scheme to at least a portion of the data points sampled from the location detecting unit 15. In some embodiment, this may include, for example, applying linear averaging or non-linear averaging to at least a portion of the location data or location signals. In some embodiments, the operations of box 1020 may include, for example, applying median smoothing or median filtering to at least a portion of the localization data or localization signals. Other filtering or smoothing operations may be performed in accordance with embodiments of the invention by data modifying unit 17.

As indicated in box 1023, the method may optionally include constructing a two dimensional tracking curve may be from data obtained from, for example the location detection unit 15. In other embodiments of the present invention, a three dimensional tracking curve or other suitable tracking curves may be constructed and displayed. The plane that may be defined by two dimensions may represent, for example, the plane where most of the movement of device 40 through for example the GI tract may occur, for example it may be coronal plane, substantially the coronal plane, or any other suitable plane. In some embodiments of the present invention, the tracking curve may be, for example, a tracking curve of device 40 in the substantially coronal plane.

As indicated in box 1027, the method may optionally include determining distances between point on the tracking curve. In some embodiments the distance determined may be the distance within the two dimensional plane or may be the distance in three dimensional space. Distances may be compared to thresholds, as may be described herein or may be used for other suitable analysis.

As indicated at box 1030, the method may optionally include, for example, applying a curve smoothing process or scheme, for example, to the tracking curve obtained, to at least a portion of the data points sampled in at least two dimensions by location detecting unit 15 in, for example, substantially real time. In some embodiments, data modification by data modification unit 17 may include, for example, applying an X-Z curve smoothing process to at least a portion of the location data or location signals. Other curve smoothing operations may be performed as may have been described herein and may be in accordance with embodiments of the invention.

In some embodiments, as indicated at box 1040, the data modification by data modification unit 17 includes, for example, processing or modifying data points sampled from location detecting unit in relation to, or based on, information sensed or imaged by an in-vivo device and/or in-vivo image sensor. For example, in some embodiments, the method may include, motility detection by motility detector 16, for example, comparing between two or more images acquired by the in-vivo imaging device, or analyzing one or more images acquired by the in-vivo imaging device. Then, analysis, it may, for example, be determined that the in-vivo imaging device did not move during the time period in which the images were acquired; and thus, location data may be updated or modified, e.g., to indicate non-movement of the in-vivo imaging device at that time period. In other embodiments image content or comparison may be used in other ways to modify location data sampled by location detecting unit 15. In other embodiments, modification of data points sampled by location detecting unit 15 may be performed prior to filtering, prior to curve smoothing, or in other suitable order.

As indicated at box 1050, the method may optionally include, for example, performing other suitable operations, e.g., storing the modified location data points or signals, printing the location data or signals, displaying the location data or signals, or otherwise processing the location data or signals.

It is noted that some or all of the above-mentioned operations may be performed substantially in real time, e.g., during the operation of the in-vivo imaging device, during the time in which the in-vivo imaging device operates and/or captures images, and/or without interruption to the operation of the in-vivo imaging device. Other operations or sets of operations may be used in accordance with embodiments of the invention.

A device, system and method in accordance with some embodiments of the invention may be used, for example, in conjunction with a device which may be inserted into a human body. However, the scope of the present invention is not limited in this regard. For example, some embodiments of the invention may be used in conjunction with a device which may be inserted into a non-human body or an animal body.

Some embodiments of the invention may be implemented by software, by hardware, or by any combination of software and/or hardware as may be suitable for specific applications or in accordance with specific design requirements. Embodiments of the invention may include units and/or sub-units, which may be separate of each other or combined together, in whole or in part, and may be implemented using specific, multi-purpose or general processors, circuits or controllers, or devices as are known in the art. Some embodiments of the invention may include buffers, registers, storage units and/or memory units, for temporary or long-term storage of data or in order to facilitate the operation of a specific embodiment.

Some embodiments of the invention may be implemented, for example, using a machine-readable medium or article which may store an instruction or a set of instructions that, if executed by a machine, for example, by device 100, by device 40, by processor 14, by data modifying unit 17, motility detector 16, location detecting unit 15 or by other suitable machines, may cause the machine to perform a method and/or operations in accordance with embodiments of the invention. Such machine may include, for example, any suitable processing platform, computing platform, computing device, processing device, computing system, processing system, computer, processor, or the like, and may be implemented using any suitable combination of hardware and/or software. The machine-readable medium or article may include, for example, any suitable type of memory unit, memory device, memory article, memory medium, storage device, storage article, storage medium and/or storage unit, for example, memory, removable or non-removable media, erasable or non-erasable media, writeable or rewriteable media, digital or analog media, hard disk, floppy disk, Compact Disk Read Only Memory (CD-ROM), Compact Disk Recordable (CD-R), Compact Disk ReWriteable (CD-RW), optical disk, magnetic media, various types of Digital Versatile Disks (DVDs), a tape, a cassette, or the like. The instructions may include any suitable type of code, for example, source code, compiled code, interpreted code, executable code, static code, dynamic code, or the like, and may be implemented using any suitable high-level, low-level, object-oriented, visual, compiled and/or interpreted programming language, e.g., C, C++, Java, BASIC, Pascal, Fortran, Cobol, assembly language, machine code, or the like.

## Claims

1. A system for tracking an in-vivo image sensor (46), the system comprising:

   a location detecting unit (15) to locate the in-vivo image sensor (46) over time;
   a data modifying unit (17) to modify data sampled by the location detecting unit (15) based on information sensed by the in-vivo image sensor(46);
   a signal strength detector (24); and
   at least one antenna (10), wherein the antenna (10) is to receive a signal transmitted from the in-vivo image sensor (46).

2. The system according to claim 1, wherein the data modifying unit (17) is to modify data in substantially real time.

3. The system according to claim 1 or 2, wherein the data modifying unit (17) comprises a median filter.

4. The system according to any one of claims 1 to 3, wherein the in-vivo image sensor (46) is to acquire in-vivo images.

5. The system according to any one of claims 1 to 4, wherein the data modifying unit (17) comprises a motility detector (16).

6. The system according to claim 5, wherein the motility detector (16) is to compare images acquired by the in-vivo image sensor (46).

7. The system according to any one of claims 1 to 6, comprising a display unit (18) to display tracking information of the in-vivo image sensor (4G).

**8.** The system according to any one of claims 1 to 7, wherein the in-vivo image sensor (46) is autonomous.

**9.** The system according to any one of claims 1 to 8, comprising a swallowable capsule (40) including the in-vivo image sensor (46).

**10.** The system according to any one of claims 1 to 8, comprising:

a swallowable capsule (40) including at least the in-vivo image sensor (46) and a transmitter (41) to transmit image data;
an antenna array (10) to receive signals transmitted from the transmitter (41); and
a recorder (19) to record the received signals.

**Patentansprüche**

**1.** System zum Nachverfolgen eines In-Vivo-Bildsensors (46), das System mit:

einer Positionserkennungseinheit (15), um den In-Vivo-Bildsensor (46) über der Zeit zu lokalisieren;
einer Datenverändetungseinheit (17), um Daten, die von der Positionserkennungseinheit (15) aufgenommen wurden, basierend auf Information zu verändern, die vom In-Vivo-Bildsensor (46) erfasst wurde;
einem Signalstärkedetektor (24); und
zumindest einer Antenne (10), wobei die Antenne (10) dafür ausgebildet ist, ein Signal zu empfangen, das von dem In-Vivo-Bildsensor (46) gesendet wurde.

**2.** System nach Anspruch 1, wobei die Datenveränderungseinheit (17) dafür ausgebildet ist, die Daten im Wesentlichen in Echtzeit zu verändern.

**3.** System nach Anspruch 1 oder 2, wobei die Datenvcränderungseinheit (17) einen Median-Filter aufweist.

**4.** System nach einem der Ansprüche 1 bis 3, wobei der In-Vivo-Bildsensor (46) dafür ausgebildet ist, In-Vivo-Bilder zu erfassen.

**5.** System nach einem der Ansprüche 1 bis 4, wobei die Datenveränderungseinheit (17) einen Bewegungsdetektor (16) aufweist.

**6.** System nach Anspruch 5, wobei der Bewegungsdetektor (16) dafür ausgebildet ist, Bilder zu vergleichen, die von dem In-Vivo-Bildsensor (46) erfasst wurden.

**7.** System nach einem der Ansprüche 1 bis 6, mit einer Anzeigeeinheit (18), um Nachverfolgungsinformation des In-Vivo-Bildsensors (46) darzustellen.

**8.** System nach einem der Ansprüche 1 bis 7, wobei der In-Vivo-Bildsensor (46) autonom ist.

**9.** System nach einem der Ansprüche 1 bis 8 mit einer schluckbaren Kapsel (40), die den In-Vivo-Bildsensor (46) aufweist.

**10.** System nach einem der Ansprüche 1 bis 8 mit:

einer schluckbaren Kapsel (40), die mindestens den In-Vivo-Bildsensor (46) und einen Sender (41) zum Übertragen von Bilddaten aufweist;
einem Antennenarray (10), um Signale zu empfangen, die von dem Sender (41) gesendet wurden; und
einer Aufnahmeeinheit (19), um die empfangenen Signale aufzunehmen.

**Revendications**

1. Système pour localiser un capteur (46) d'image in-vivo, le système comprenant :

   - une unité (15) de détection de position pour localiser un capteur (46) d'image in-vivo, en fonction du temps ;
   - une unité (17) de modification de données pour modifier des données échantillonnées par l'unité (15) de détection de position, basée sur l'information captée par le capteur (46) d'image in-vivo ;
   - un détecteur (24) de force de signal ; et
   - au moins une antenne (10), apte à recevoir un signal transmis depuis le capteur (46) d'image in-vivo.

2. Système selon la revendication 1, **caractérisé en ce que** l'unité (17) de modification de données est apte à modifier les données sensiblement en temps réel.

3. Système selon l'une des revendications 1 ou 2, **caractérisé en ce que** l'unité (17) de modification de données comporte un filtre médian.

4. Système selon l'une des revendications 1 à 3, **caractérisé en ce que** le capteur (46) d'image in-vivo est apte à acquérir des images in-vivo.

5. Système selon l'une des revendications 1 à 4, **caractérisé en ce que** l'unité (17) de modification de données comporte un détecteur (16) de motilité.

6. Système selon la revendication 5, **caractérisé en ce que** le détecteur de motilité (16) est apte à comparer des images acquises par le capteur (46) d'image in-vivo.

7. Système selon l'une des revendications 1 à 6, **caractérisé en ce qu'**il comporte une unité (18) d'affichage pour afficher l'information de localisation du capteur (46) d'image in-vivo.

8. Système selon l'une des revendications 1 à 7, **caractérisé en ce que** le capteur (46) d'image in-vivo est autonome.

9. Système selon l'une des revendications 1 à 8, **caractérisé en ce qu'**il comporte une capsule (40) susceptible d'être avalée incluant le capteur (46) d'image in-vivo.

10. Système selon l'une des revendications 1 à 8, comprenant :

    - une capsule (40) susceptible d'être avalée incluant au moins le capteur (46) d'image in-vivo et un transmetteur (41) pour transmettre des données d'images ;
    - un réseau d'antenne (10) pour recevoir des signaux transmis depuis le transmetteur (41) ; et
    - un enregistreur (19) pour enregistrer les signaux reçus.

FIG.1A

FIG.1B

10a    ° ° °    10z

25 — ANTENNA
SELECTOR

22 — RECEIVER

20

DATA STORAGE — 22

SIGNAL STRENGTH
MEASUREMENT UNIT — 24

PROCESSING
UNIT — 26

FIG.2

FIG.3

FIG.4

FIG.5

FIG.6

FIG.7

FIG.8

FIG.9A

FIG.9B

RECEIVE  LOCALIZATION  DATA —1010

APPLY  SMOOTHING / FILTERING —1020

CONSTRUCT  A  2D  TRACKING  CURVE —1023

DETERMINE  DISTANCE  BETWEEN  POINTS —1027

APPLY  CURVE  SMOOTHING —1030

MODIFY  LOCALIZATION  DATA
BASED  ON  SENSED  INFORMATION —1040

PERFORM  OTHER  OPERATIONS —1050

FIG.10

**EP 1 676 522 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0667115 A
- US 09800470 B
- US 5604531 A
- US 10046541 B
- US 11073633 B
- US 6904308 B
- US 10150018 B
- US 20020173718 A
- US 6709387 B